Europäisches Patentamt

(19) European Patent Office

Office européen des brevets

(11) EP 0 958 822 A1

(12) **EUROPEAN PATENT APPLICATION**
published in accordance with Art. 158(3) EPC

(43) Date of publication:
24.11.1999 Bulletin 1999/47

(21) Application number: 97925820.9

(22) Date of filing: 13.06.1997

(51) Int. Cl.$^6$: **A61K 31/55**, A61K 31/40

(86) International application number:
PCT/CN97/00060

(87) International publication number:
WO 98/01137 (15.01.1998 Gazette 1998/02)

(84) Designated Contracting States:
BE CH DE ES FR GB IT LI SE

(30) Priority: 10.07.1996 CN 96107042

(71) Applicant: Lin, Tongho
Taipei, Taiwan (CN)

(72) Inventors:
• LIN, Tongho
Taipei, Taiwan (CN)

• CHEN, Ingjun
Kaohsiung, Taiwan (CN)

(74) Representative:
DIEHL GLAESER HILTL & PARTNER
Patentanwälte
Königstrasse 28
22767 Hamburg (DE)

(54) **THE OCULAR HYPOTENSIVE ACTIVE COMPOSITIONS**

(57) The present invention is related to the ocular hypotensive active compositions, which comprise clozapine or sulpiride and pharmaceutically acceptable excipients. The ocular preparations of the invention have ocular hypotensive effect (10P). They are new drugs in the therapy of glaucoma which can be easily prepared, and are so safe in clinical practice that no side-effect has been found.

## Description

*Field of the invention*

The title of the invention is " The ocurlar hypotensive active compositions", concerns the pharmaceutical field.

*Background of the invention*

According to the glaucoma disease is suffered easily for human and none efficacious drug. Just by the methods of reduce intraocular pressure(IOP) and increase the blood flow to the retina that have barely curative effect.

In C. Y. George's 1989 experiment, it was discovered that some dopamine receptor antagonists belonging to an antipsychotic drug were found to be able to reduce intraocular pressure(IOP) and increase the blood flow to the retina, choroid, iris and ciliary body in rabbits. According to some reports, the antipsychotic drugs can be generally categorized to be dopamine receptor antagonist. Those drug which will produce side effects of parkinsonism and tarditive dyskinesia.

As other student reported that a non selective β-adrenergic receptors L-timolol could treat patients with glaucoma in clinics. Therefore it was thought that β blockers drugs could lower the IOP in treating patients with glaucoma. Then in **1986** Inv. Ophthalmol. Vis. Sci. Vol. 27 reported that L-timolol couldn't improve parcipitation of retinopathy. This fact has led many investigators to search for a newer anti-glaucoma agent.

*Detail descriptions of the invention*

The present invention is aimed at discovering a new anti-glaucoma agent which have the character of reduce intraocular pressure(IOP), increase the blood flow to the retina, and less side effects. There is a safe agent in clinics

This invention reveals a novel anti-glaucoma solution which comprises of clozapine or sulpiride and a pharmaceutically carrier. For treatment, various saline, diluents, lubricants, stabilizer and binders are added with clozapine and sulpiride. Alkaline mineral acid is used to adjust the pH balance to prepare ophthalmic ointment and solution.

In this invention Clozapine and Sulpiride have been used to prepare the ophthalmic ointment and solution. Both emulsified and non-emulsified clozapine had the same treatment result in decreasing IOP in animals. Mammals were mainly used in this experiment. The general dosage depends on the symptom. Usually for each person one to three drops each time, three times a day.

In general, Clozapine drop solution is prepared first by dissolving clozapine in 0.9% NaCl solution, and Sulpiride is mixed by propylene glycol. Then the pH balance is adjusted with mineral acid. Later a stabilizer or emulsed stabilizer is added. This invention reveals an anti-glaucoma medicinal combination which comprises of Clozapine and Sulpiride. It is optional to prepare a ophthalmic drop solution or ointment. Ophthalmic solution is made of NaCl, boric acid, alkaline mineral acid and sodium sulphate. Or alkaline is used to compose pH 7.4 $PO_4$ saline The alkaline mineral acids used were $Na_2HPO_4$ and $NaH_2PO_4$. If necessary, a stabilizer or fungicide could be added. Choose carboxymethycellulose, hydroxypropyl β cyclodextrin to be the stabilizer; and chlorobutanol to be the fungicide. Usually choose non-irritant white Vaseline, mineral oil or water-free sheep oil to be the base ingredient of the ointment.

*Pharmacological activity and experiment method*

(1.) The experiment method used to research the decreasing of IOP activity in clozapine and sulpiride followed the reports in Vareilles **1981**, vol 13 pp72-79. New Zealand albino rabbits of either sex were used in the study. A 20 % hypertonic saline solution was infused at the speed of 1 ml per mm for 10 minutes through the marginal ear vein with an infusion pump. After dropping 0.5% alcaine eye drops in both eyes for one minute, Clozapine drop solution (50 μl, 0.5%) and vehicle drop solution(50 μl) were gradually instilled respectively.

Then the IOP of both eyes were measured with a Tono-Pen XL Tonometer (Mentor, Norwell , MA) at 30 minute and 10 minute intervals before the experiment. At the time of commencement, as well as at 10, 20, 40, 60, and 80 min intervals measurements were taken. Then measurements were repeated at every 30 minute intervals. Identical experiments were repeated for another group of animals with emulsified Clozapine drop solution (50 μl, 0.5%) and Sulpiride drop solution (50 μl, 20%).

The IOP recovery curve acquired had shifted to the right and below the curve of the controlled group. It indicated that these drugs have the activity to decrease IOP.

Results

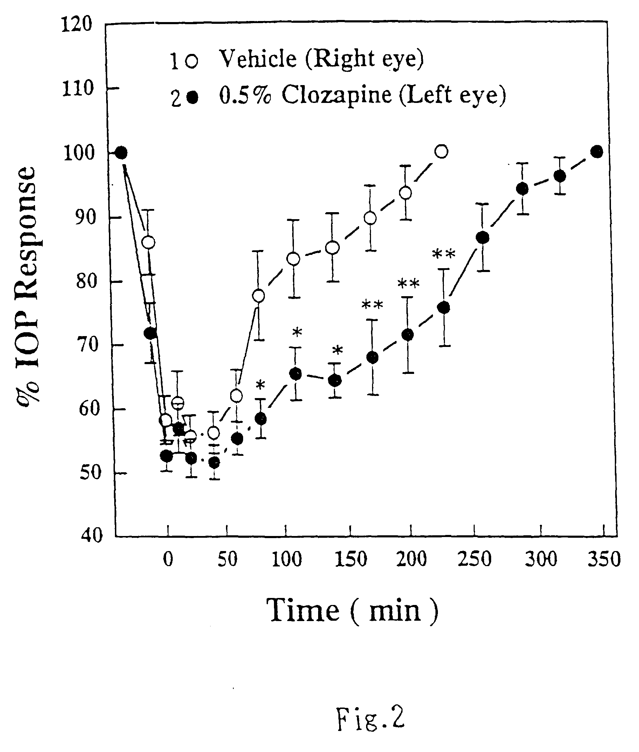

IOP is considered to be the major factor which causes glaucoma, and drugs with activity of reducing IOP are considered to be able to treat glaucoma. As shown in Fig.2, 0.5% Clozapine drop solution was able to decrease the IOP recovery time of rabbits. It inhibited the IOP response more than the control group.

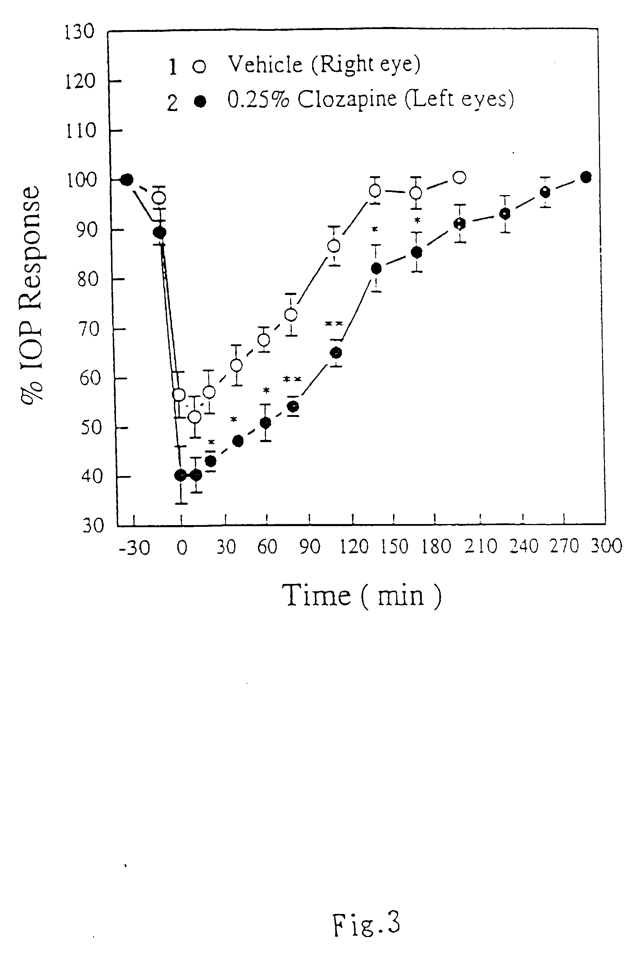

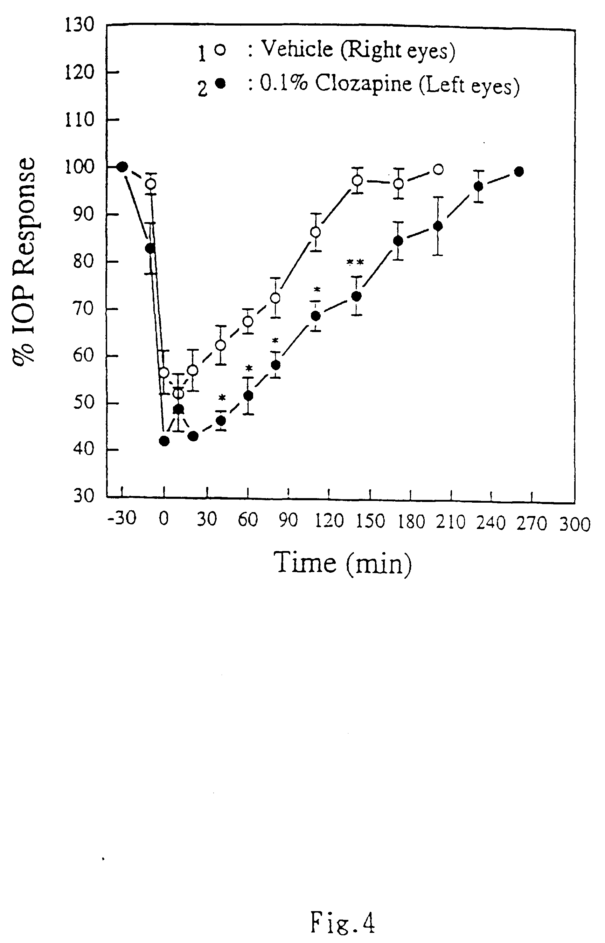

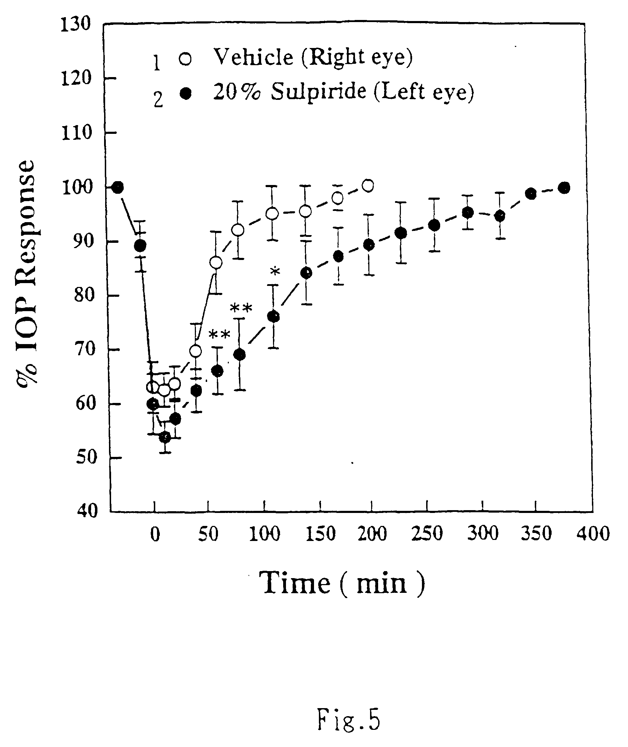

As shown in Fig.3 and Fig.4, 0.25% and 0.1% dissolved Clozapine drop solution showed that it decreased IOP recovery time of rabbits more mildly. Although 0.5% Sulpiride didn't show a significant ability in decreasing IOP, it did so when the concentration was raised to 20% as shown in Fig.5.

(2) Measurement of the vasodilatation effect

A stunned guinea pig of 350-400g was taken and an incision made at the arched place near the main artery. Then the main artery and all relevant tissue were taken out from the chest. They were put into saline immediately then cut into lengths of 2-4 MM. The main artery loop was installed on a parallel hook with 1g tensile strength inside an organ container full of 10ml saline. The composition of the saline in the container was NaCl 112 mM, KCl 5.0Mm, $MgSO_4$ 1.2mM, $NaHCO_3$ 25mM, $KH_2PO_4$ 1.0mM, $CaCl_2$ 1.25mM and glucose 11.5mM. The organ container was kept at a temperature of 37°C. Then 95% oxygen and 5% carbon dioxide were mixed in.

The strength shown on the transducer was used as the tensile strength of the main artery loop. Before the experiment could be started, all the equipment had to be soaked in saline for one hour. The first procedure was to test the againsting $\alpha$ - adrenaline function, so as to test the biggest muscle contraction function of $10^{-6}$ M phenylephrine. Clozapine was used to release its contraction.

According to Godfraing's report in **1969** Br. J. Pharmacol vol. 36, 549-560, depolarizing solution of high concentrated $k^+$ would cause the main artery's contraction. The Depolarizing solution of high concentrated $k^+$ was composed of NaCl 17mM, KCl 100mM, $MgSO_4$ 1.2mM, $NaHCO_3$ 25mM, $KH_2PO_4$ 1.0mM, $CaCl_2$ 1.25mM and glucose 11.5mM. Clozapine was added to the organ container when the biggest contraction occurred, which reduced the main artery's contraction.

Result

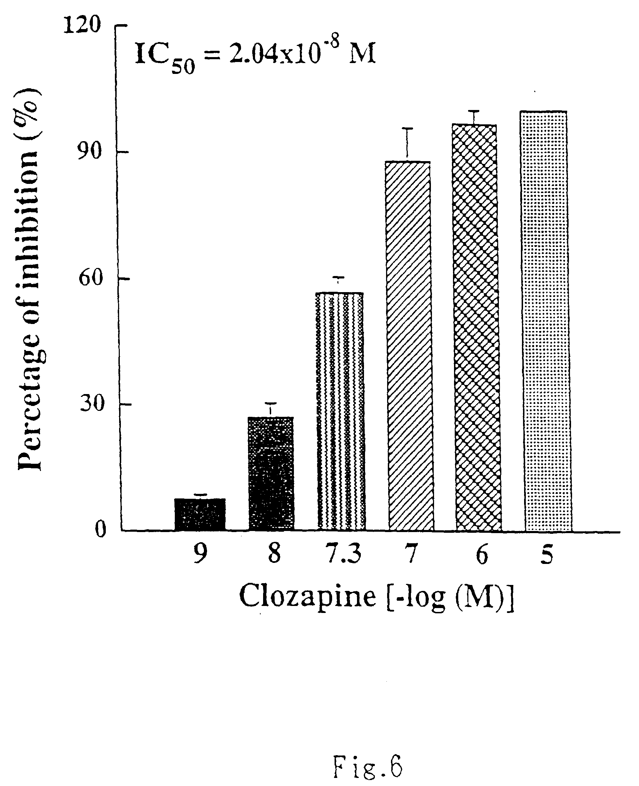

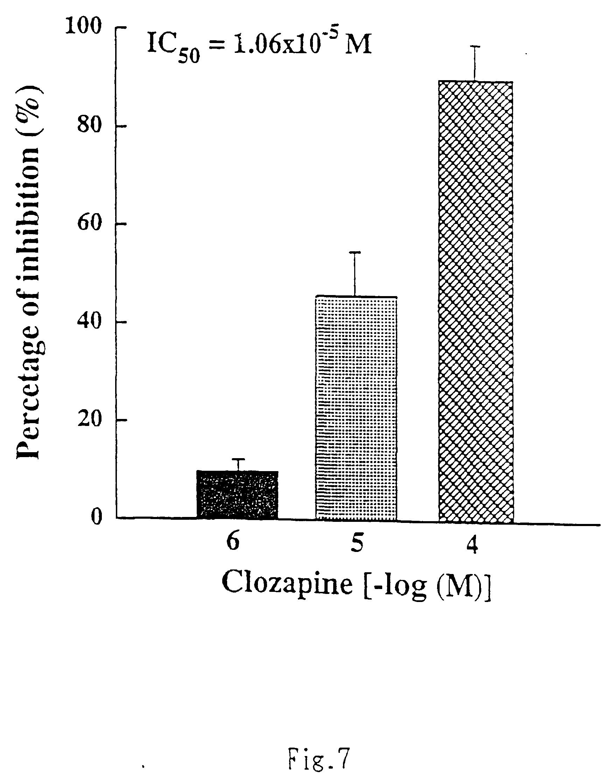

The test result of the vasodilation effect is shown as Fig 6. When the concentration of Clozapine increases, it could effectively restrain contraction caused by phenylephrine. The estimated $IC_{50}$ value is 2 $\times 10^{-8}$ M. Clozapine also could restrain contraction caused by $K^+$, restrain the calculated contraction caused by Ca. As the $IC_{50}$ value shown in Fig.7, Clozapine could restrain the constriction caused by phenylephrine.

(3) Clozapine increases ocular blood flow

The experiment format of increasing blood flow used was following the method what Chiou, G. C. Y & Co. had reported in **1993** J. Ocular Pharmacol vol.9 pp.179-185.

We chose New Zealand white rabbits weighing 2.5-3.0 kg anesthetized by injecting 35mg/kg ketamine and 5 mg/kg xylazine. Half of the initial dose was given after one hour to maintain adequate anesthesia. The left ventricle was cannulated through the right carotid artery to inject colored microspheres (the diameter was 15) , and the femoral artery was cannulated for blood collection.0.1% and 0.25% Clozapine solution or vehicle were instilled in the eyes. The ocular blood flow of a ocular hypertensive rabbit was measured with colored microspheres at 0, 30, 60, 120, and 180 min intervals, thereafter. After one minute of each injection of microspheres, blood samples were taken from the femoral artery immediately as a reference. The blood samples were collected in a tube with heparin and the volume of each was recorded.

After injecting the last microspheres and collecting the last blood samples, the animals were sacrificed and the eyes were dissected. The retina, choroid, iris and ciliary body were all weighted.

The samples were processed by an American company E-Z Trac and the counting method of microsphere was provided by them too. Tissue samples were put in microfuge tubes with tissue (blood) digest reagent I, and the caps were sealed tightly. They were heated at 95°C for 15 minutes, then vortexed in a vortexer for 30 minutes. The tissue samples were heated and vortexed until they were completely dissolved. While the tissue samples were still with heat, they were added with Tissue (Blood) Digest Reagent II, and the caps were sealed. They were vortexed and the tubes were centrifuged to settle the microspheres to the bottom of the tubes. Then the supernatant was removed and the pellet was resuspended in a Microsphere Counting Reagent of precise volume. The number of various colored microspheres were counted with an emocytometer.

Hemolysis Reagent was added to the blood samples. Then they were vortexed and centrifuged for 30 minutes in a low revolution, Supernatant was removed, and Tissue (Blood) Digest Reagent I was added. As followed in the previously stated procedure to test tissue samples, the number of colored microspheres were counted.

The blood flow of each tissue at a certain time point was able to be calculated with the following equation: $Qm=(Cm \times Qr)/Cr$

Qm : the blood flow of a tissue, the unit is $\mu l/min/mg$
Cm : the total number of tissue microsphere per mg
Qr : the flow rate of blood sample, the unit is $\mu l/mm$
Cr : the total number of microsphere of referenced blood sample.

Result

As shown in Table 1 of ocular blood flow in retina, choroid, iris and ciliary body. No matter if the clozapine was of 0.1, 0.25% or 0.5.% they showed significant increases in ocular blood flow from 30 to 180 minutes.

*Brief description of the drawings*

Table 1    Clozapine increases ocular blood flow

    1. iris,
    2. ciliary body
    3. retina
    4. choroid

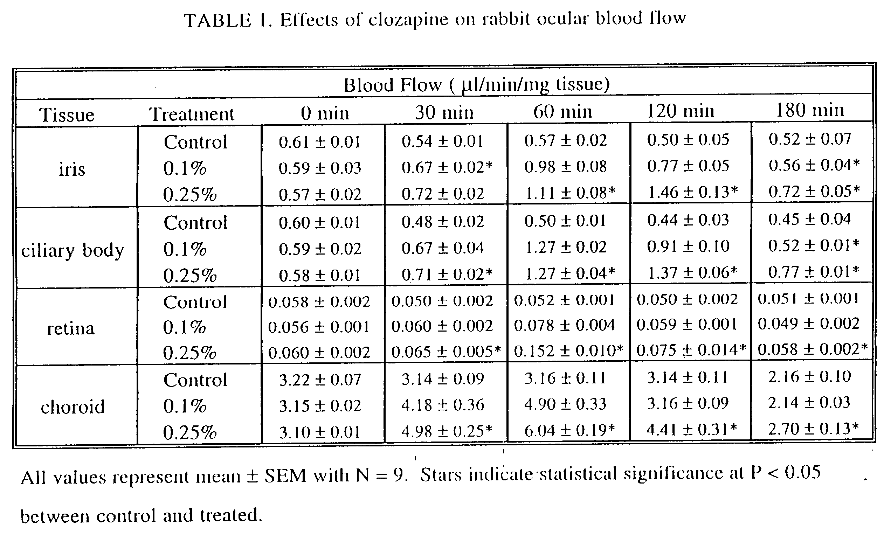

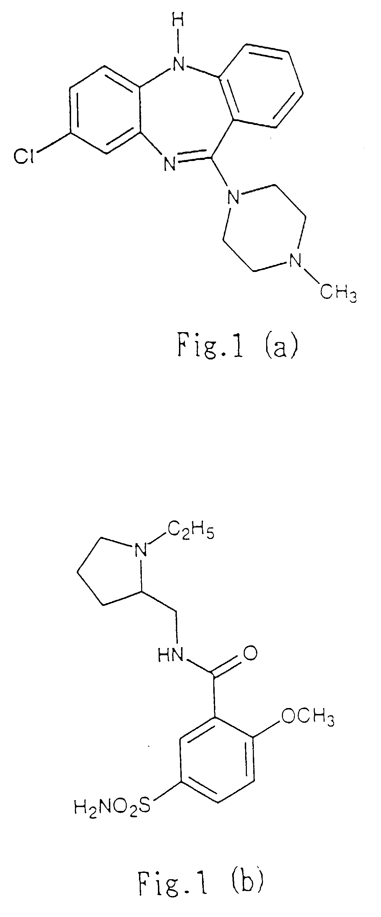

Fig. 1    chemical structure
    (a) Clozapine    (b) Sulpiride
Fig. 2    Clozapine was able to decrease IOP response in rabbits.
    1. comparison( right eye)    2. 0.5% Clozapine (left eye)
Fig. 3    Clozapine was able to decrease IOP response in rabbits.
    1. comparison(right eye)    2. 0.25% Clozapine(left eye)
Fig. 4    Clozapine was able to decrease IOP response in rabbits.
    1. comparison(right eye)    2. 0.1% Clozapine(left eye)
Fig. 5    1. comparison (right eye)    2. 20% Sulpiride (left eye)
Fig.6    Clozapine's restraint ratio to phenylephrine
Fig.7    Clozapine's restraint ratio to phenylephrine

*Optimum Example of the invention*

**Example 1**    Clozapine drop solution

    20% Clozapine
    0.9% NaCl
    dilute $Na_2HPO_4$
    1.5% hydroxypropyl β -cyclodextrin carboxymethycellulose

**Example 2**    Sulpiride drop solution

    20% Sulpiride
    propyleneglycol (20%)
    0.9% NaCl

**Example 3**    Clozapine drop solution

    0.25% Clozapine
    0.9 NaCl
    dilute HCl

**Example 4**    Clozapine drop solution

    0.1% Clozapine
    0.9 NaCl
    dilute HCl

**Claims**

1.    An IOP pharmaceutical composition comprising primarily ingredient of Clozapine with a pharmaceutical vehicle.

2.    An IOP pharmaceutical composition comprising primarily ingredient sulpiride with a pharmaceutical vehicle.

3.    An IOP compositions as claimed in claim 1 with an adjusted pH value with saline to prepare ocular ointment and drop solution.

TABLE 1. Effects of clozapine on rabbit ocular blood flow

| | | Blood Flow ( $\mu$l/min/mg tissue) | | | | |
|---|---|---|---|---|---|---|
| Tissue | Treatment | 0 min | 30 min | 60 min | 120 min | 180 min |
| iris | Control | 0.61 ± 0.01 | 0.54 ± 0.01 | 0.57 ± 0.02 | 0.50 ± 0.05 | 0.52 ± 0.07 |
| | 0.1% | 0.59 ± 0.03 | 0.67 ± 0.02* | 0.98 ± 0.08 | 0.77 ± 0.05 | 0.56 ± 0.04* |
| | 0.25% | 0.57 ± 0.02 | 0.72 ± 0.02 | 1.11 ± 0.08* | 1.46 ± 0.13* | 0.72 ± 0.05* |
| ciliary body | Control | 0.60 ± 0.01 | 0.48 ± 0.02 | 0.50 ± 0.01 | 0.44 ± 0.03 | 0.45 ± 0.04 |
| | 0.1% | 0.59 ± 0.02 | 0.67 ± 0.04 | 1.27 ± 0.02 | 0.91 ± 0.10 | 0.52 ± 0.01* |
| | 0.25% | 0.58 ± 0.01 | 0.71 ± 0.02* | 1.27 ± 0.04* | 1.37 ± 0.06* | 0.77 ± 0.01* |
| retina | Control | 0.058 ± 0.002 | 0.050 ± 0.002 | 0.052 ± 0.001 | 0.050 ± 0.002 | 0.051 ± 0.001 |
| | 0.1% | 0.056 ± 0.001 | 0.060 ± 0.002 | 0.078 ± 0.004 | 0.059 ± 0.001 | 0.049 ± 0.002 |
| | 0.25% | 0.060 ± 0.002 | 0.065 ± 0.005* | 0.152 ± 0.010* | 0.075 ± 0.014* | 0.058 ± 0.002* |
| choroid | Control | 3.22 ± 0.07 | 3.14 ± 0.09 | 3.16 ± 0.11 | 3.14 ± 0.11 | 2.16 ± 0.10 |
| | 0.1% | 3.15 ± 0.02 | 4.18 ± 0.36 | 4.90 ± 0.33 | 3.16 ± 0.09 | 2.14 ± 0.03 |
| | 0.25% | 3.10 ± 0.01 | 4.98 ± 0.25* | 6.04 ± 0.19* | 4.41 ± 0.31* | 2.70 ± 0.13* |

All values represent mean ± SEM with N = 9. Stars indicate statistical significance at $P < 0.05$ between control and treated.

EP 0 958 822 A1

Fig.1 (a)

Fig.1 (b)

Fig.2

Fig.3

Fig.4

Fig.5

$IC_{50} = 2.04 \times 10^{-8}$ M

Fig.6

Fig.7

| INTERNATIONAL SEARCH REPORT | International application No. |
|---|---|
| | PCT/CN 97/00060 |

**A. CLASSIFICATION OF SUBJECT MATTER**

IPC⁶ A61K31/55, 31/40

According to International Patent Classification(IPC) or to both national classification and IPC

**B. FIELDS SEARCHED    A61K**

Minimum documentation searched(classification system followed by classification symbols)

IPC⁶ A61K31/55, 31/40

Documentation searched other than minimum documentation to the extent that such documents are included in the field searched

CNPAT

Electronic data base consulted during the international search(name of data base and, where practicable, search terms used)

STN  WPI  CPRS  CIPIS

**C. DOCUMENTS CONSIDERED TO BE RELEVANT**

| Category* | Citation of document, with indication, where appropriate, of the relevant passages | Relevant claim No. |
|---|---|---|
| X | WO81/03421 ( UYGE-UNIV  GEORGIA ) 10  December 1981 ( 10. 12. 81 )  See abstract | 1,3 |
| X | WO85/02114 ( ETHY-SOC ANON THPHARM;DEBR/DEBREGEAS P ) 23 May 1985 ( 23. 05. 85 )  See abstract | 2 |
| A | J Auton Pharmacol,Sep 1984,4(3)p185-92 ( Burke JA;Chang FW;Potter DE ) September 1984 ( 09. 84 ) " See abstract | 2 |

☐ Further documents are listed in the continuation of Box C.    ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason(as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 0 9 SEP 1997 | 1 8 SEP 1997 |

| Name and mailing address of the ISA/ The Chinese Patent Office 6, Xitucheng Road, Haidian District, Beijing. 100088, China | Authorized officer Jiang Hui |
|---|---|
| Facsimile No.        86-10-62019451 | Telephone No.    ( 010 ) 62093892 |

Form PCT/ISA/210(second sheet)(July 1992)

**INTERNATIONAL SEARCH REPORT**

International application No.

PCT/CN 97/00060

| Box I. | Observations where certain claims were found unsearchable(Continuation of item 1 of first sheet) |
| --- | --- |

This international search report has not been established in respect of certain claims under Article 17(2)(a)for the following reasons:

1. ☐ Claims Nos.:＿＿＿＿
because they relate to subject matter not required to be searched by Authority, namely:

2. ☐ Claims Nos.:＿＿＿＿
because they relate to parts of the international application that do not comply with the prescribed requirements to such an extent that no meaningful search can be carried out, specifically:

3. ☐ Claims Nos.:＿＿＿＿
because they are dependent claims and are not drafted in accordance with the second and third sentences of Rule 6.4(a).

| Box II. | Observations where unity of invention is lacking(Continuation of item 2 of first sheet) |
| --- | --- |

This International Searching Authority found multiple inventions in this international application, as follows:

Clozapine and sulpiride are diferent kinds of drugs,which lack same or related technical features of the invetion.

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims: it is covered by claims Nos.:

**Remark on Protest**　　☐ The additional search fees were accompanied by the applicant's protest.

☒ No protest accompanied the payment of additional search fees.

Form PCT/ISA/210(continuation of first sheet(1))(July 1992)